# EUROPEAN PATENT SPECIFICATION

(11) **EP 1 553 410 B1**
(45) Date of publication and mention of the grant of the patent: **08.06.2011**
(21) Application number: 04030954.4
(22) Date of filing: 29.12.2004
(51) Int. Cl.: G01N 33/487, G01N 15/02, G01N 33/53, G01N 33/543

(54) **Apparatus for immunoassays and application thereof**
Vorrichtung zur Durchführung immunoanalytischer Verfahren und Verfahren zu ihrer Anwendung
Dispositif de dosages immunoanalytiques et procédé pour son utilisation

(30) Priority: 07.01.2004 JP 2004002390
(43) Date of publication of application: 13.07.2005
(73) Proprietor: SYSMEX CORPORATION, Kobe-shi, Hyogo 651-0073 (JP)
(72) Inventor: Kawate, Yasunori, Kakogawa-shi Hyogo 675-0104 (JP); Matsumoto, Teruya, Kako-gun Hyogo 675-1126 (JP); Otsubo, Kayoko, Kobe-shi Hyogo 654-0006 (JP)
(74) Representative: HOFFMANN EITLE

(56) References cited:
- EP-A- 1 365 240
- EP-A- 1 387 171
- US-A- 4 284 412
- US-A1- 2003 082 662
- US-B1- 6 200 820
- US-B1- 6 228 652

## Description

### BACKGROUND OF THE INVENTION

### 1. Field of the Invention

The present invention relates to an immunoassay apparatus and an immunoassay method, and more particularly to an apparatus and a method for detecting and analyzing an analyte contained in a specimen such as blood or urine by immunological aggregation reaction using carrier particles.

### 2. Description of the Related Art

Immunoassay methods of detecting an analyte contained in a specimen such as blood using antigen-antibody reaction are widely used in the field of clinical tests. The particle aggregation method is one of such immunoassay methods. The particle aggregation method is a method of detecting an analyte by using antigen-antibody reaction, and uses carrier particles on which an antibody or an antigen against the analyte is immobilized. In the particle aggregation method, a specimen is mixed with carrier particles on which an antibody or an antigen is immobilized so as to generate aggregation of the carrier particles by antigen-antibody reaction, and the analyte contained in the specimen is detected by measuring the aggregation.

Generally, the specimen used in the above-described particle aggregation method is serum or plasma. This is because spurious particles such as blood cell components such as erythrocytes and platelets that are present in blood, fragments of the blood cell components such as fractured erythrocytes and fractured platelets, and fat particles give influence on the detection of carrier particle aggregation. For this reason, the blood obtained from a person to be tested must pass through work such as centrifugation so as to prepare serum or plasma from the whole blood.

However, when serum and plasma are used as a specimen, spurious particles having a comparatively small size such as fat particles contained in whole blood sometimes cannot be completely removed even through the work such as centrifugation. In such a case, spurious particles remaining in the serum and plasma may possibly affect the detection of aggregation. Also, when one wishes to obtain measurement results quickly such as in the case of emergency test, a measurement method is desired that uses whole blood as a specimen and eliminates the need for preparation of serum and plasma which is cumbersome and time consuming.

As an immunoassay method by the particle aggregation method that can make an accurate measurement even with the use of a specimen containing spurious particles, an immunoassay method using a technique disclosed in Unites States Patent No. 5,527,714 is known. The aforementioned United States Patent discloses a particle size distribution preparing method of inferring the particle size distribution of spurious particles in a counting immunoassay (CIA) using carrier particles, and making a correction by subtracting the inferred particle size distribution of the spurious particles from the particle size distribution containing the carrier particles and the spurious particles.

In a CIA using carrier particles, first a specimen containing an analyte is mixed with carrier particles on which an antibody or an antigen against the analyte is immobilized, so as to let the carrier particles aggregate by antigen-antibody reaction. Then, the aggregation is optically detected to obtain a particle size distribution of the carrier particles, and the degree of aggregation of the carrier particles is analyzed from the particle size distribution to examine the concentration of the analyte. However, by this method, if spurious particles are present in the specimen, the distribution of the spurious particles appears in the particle size distribution diagram of the carrier particles. Therefore, by using a particle size distribution preparing method such as disclosed in the aforementioned United States Patent, the influence of the spurious particles can be removed. This particle size distribution preparing method infers the particle size distribution of the spurious particles by interpolation with a spline function, and makes a correction by subtracting the inferred particle size distribution of the spurious particles from the particle size distribution containing the carrier particles and the spurious particles. By this correction, one can obtain the particle size distribution diagram of the carrier particles from which the influence of the spurious particles is removed. Thus, a measurement result having a clinically sufficiently high precision can be obtained even in the case of using a specimen containing spurious particles.

Both EP1 387 171 A (which is a document of the state of the art pursuant to Article 54(3) EPC only), as well as EP1 365 240 A, disclose a method and an apparatus for immunoagglutination assays in which particle fluorescence is used as an optical information which is independent of particle size.

However, in the field of research and others, a measurement result having a higher precision is sometimes required.

### SUMMARY OF THE INVENTION

The present invention solves the aforementioned problems, and provides immunoassay apparatus and method that can yield a measurement result having a higher precision than in the prior art.

A first aspect of the present invention relates to an immunoassay apparatus comprising: (a) a measuring sample preparing section for preparing a measuring sample by mixing a specimen with carrier particles on which an antibody or an antigen against an analyte is immobilized; (b) a detector for detecting internal information and size information from particles contained in the measuring sample which internal information is selected from the group consisting of side scattered light and high frequency current resistance; and (c) a controller which identifies the carrier particles on the basis of the obtained internal information and calculates a degree of aggregation of the identified carrier particles on the basis of the obtained size information.

A second aspect of the present invention relates to an immunoassay apparatus comprising: (a) a measuring sample preparing section for preparing a measuring sample by mixing a specimen with carrier particles on which an antibody or an antigen against an analyte is immobilized; (b) a detector for detecting side scattered light and forward scattered light from particles contained in the measuring sample; and (c) a controller for identifying the carrier particles on the basis of the detected side scattered light and for calculating a degree of aggregation of the identified carrier particles on the basis of the detected forward scattered light.

A third aspect of the present invention relates to a method of identifying carrier particles in an immunoassay, comprising the steps of: (a) preparing a measuring sample by mixing a specimen with carrier particles on which an antibody or an antigen against an analyte is immobilized; (b) detecting internal information from particles contained in the prepared measuring sample which internal information is selected from the group consisting of side scattered light and high frequency current resistance; and (c) identifying the carrier particles on the basis of the obtained internal information.

A fourth aspect of the present invention relates to a immunoassay method comprising the steps of: (a) preparing an measuring sample by mixing a specimen with carrier particles on which an antibody or an antigen against an analyte is immobilized; (b) detecting internal information and size information from particles contained in the measuring sample which internal information is selected from the group consisting of side scattered light and high frequency current resistance; (c) identifying the carrier particles on the basis of the obtained internal information; and (d) calculating a degree of aggregation of the carrier particles identified by said step (c) on the basis of the obtained size information.

A fifth aspect of the present invention relates to a immunoassay method comprising the steps of: (a) preparing a measuring sample by mixing a specimen with carrier particles on which an antibody or an antigen against an analyte is immobilized; (b) detecting side scattered light and forward scattered light from particles contained in the measuring sample; (c) identifying the carrier particles on the basis of the detected side scattered light; and (d) calculating a degree of aggregation of the carrier particles identified by said step (c) on the basis of the detected forward scattered light.

### BRIEF DESCRIPTION OF THE DRAWINGS

Fig. 1 is a view describing an outlook of an immunoassay apparatus according to one embodiment of the present invention.
Fig. 2 is a view describing an inner configuration of an immunoassay apparatus according to one embodiment of the present invention.
Fig. 3 is a view describing a measuring sample preparing section of an immunoassay apparatus according to one embodiment of the present invention.
Fig. 4 is a view describing a measuring section of an immunoassay apparatus according to one embodiment of the present invention.
Fig. 5 is a view describing a sheath flow cell part of an immunoassay apparatus according to one embodiment of the present invention.
Fig. 6 is a view describing a relationship between a controlling section and each apparatus part of an immunoassay apparatus according to one embodiment of the present invention.
Fig. 7 is a view describing a flow of overall control in an immunoassay apparatus according to one embodiment of the present invention.
Fig. 8 is a view describing a flow of an analyzing process according to one embodiment of the present invention.
Fig. 9 is a model view illustrating one example of a two-dimensional scattergram prepared by an immunoassay apparatus according to one embodiment of the present invention.
Fig. 10A is a model view illustrating one example of a histogram prepared by an immunoassay apparatus according to one embodiment of the present invention.
Fig. 10B is a model view illustrating one example of a histogram prepared by an immunoassay apparatus according to one embodiment of the present invention.
Fig. 11 is a view illustrating one example of a two-dimensional scattergram prepared by an immunoassay apparatus according to one embodiment of the present invention.
Fig. 12 is a view illustrating one example of a two-dimensional scattergram prepared by an immunoassay apparatus according to one embodiment of the present invention.
Fig. 13A is a view illustrating one example of a histogram prepared by an immunoassay apparatus according to one embodiment of the present invention.
Fig. 13B is a view illustrating one example of a histogram prepared by an immunoassay apparatus according to one embodiment of the present invention.
Fig. 14A is a view illustrating one example of a histogram prepared by an immunoassay apparatus according to one embodiment of the present invention;
Fig. 14B is a view illustrating one example of a histogram prepared by an immunoassay apparatus according to one embodiment of the present invention.
Fig. 15 is a view describing a flow of an analyzing process in an immunoassay apparatus according to another embodiment of the present invention.
Fig. 16 is a view describing a flow of an analyzing process in an immunoassay apparatus according to another embodiment of the present invention.
Fig. 17A is a view illustrating one example of a histogram prepared by an immunoassay apparatus according to one embodiment of the present invention.
Fig. 17B is a view illustrating one example of a histogram prepared by an immunoassay apparatus according to one embodiment of the present invention.

### DESCRIPTION OF THE PREFERRED EMBODIMENTS

Hereafter, an immunoassay apparatus according to one embodiment of the present invention will be described with reference to the attached drawings. First, the immunoassay apparatus prepares a measuring sample by mixing a specimen, a carrier particle suspension, and a reaction buffer solution. Then, from each particle in the prepared measuring sample, the "internal information of the particle" and the "size information of the particle" are detected so that the carrier particles are differentiated from the spurious particles on the basis of each detected information, and further the degree of aggregation of the carrier particles is calculated.

Here, the carrier particle suspension is a suspension obtained by suspending carrier particles, on which an antibody or an antigen against an analyte is immobilized, into a suitable liquid such as water or a buffer solution. When an analyte is present in a specimen, aggregation of carrier particles occurs by antigen-antibody reaction when a carrier particle suspension is added to the specimen. Here, the carrier particles may be those that are generally used in the particle aggregation method, such as latex particles, metal particles, and dendrimers. Further, regarding the antibody or antigen that immobilized on the carrier particles, when the analyte is an antibody, an antigen that undergoes an antigen-antibody reaction specifically with the antibody is used, whereas when the analyte is an antigen, an antibody that undergoes an antigen-antibody reaction specifically with the antigen is used. For example, if the measurement item is a carcinoembryonic antigen (CEA antigen), an anti-CEA antibody is immobilized on the carrier particles.

The reaction buffer solution is added together with the carrier particle suspension to the specimen so as to provide an environment that generates the antigen-antibody reaction.

Further, the "internal information of the particle" (hereinafter referred to as internal information) may be, for example, the "density within the particle" (hereinafter referred to as density). The density of the carrier particles is larger than the density of the spurious particles such as erythrocytes, platelets, and fat particles contained in the specimen. Therefore, by detecting the information that reflects the density of the carrier particles and the density of the spurious particles, the carrier particles can be differentiated from the spurious particles on the basis of the information. The information that reflects the density may be, for example, optical information such as side scattered light intensity. Further, instead of the optical information, one can use, for example, electrical information such as high frequency resistance that is obtained when particles are let to pass between the electrodes through which a high frequency current is flowing. In this embodiment, side scattered light intensity is used as the information that reflects the density.

When non-aggregated carrier particles (hereinafter referred to as single particles) and an aggregation mass formed by aggregation of a plurality of carrier particles (hereinafter referred to as aggregated particles) are compared, the aggregated particles have a larger apparent size. For this reason, by detecting the "size information of the particle" (hereinafter referred to as size information), the single particles and the aggregated particles can be differentiated and separately counted, whereby the degree of aggregation of the carrier particles can be determined. The size information may be, for example, optical information such as forward scattered light intensity. Further, instead of the optical information, one can use, for example, electrical information such as direct current resistance that is obtained when particles are let to pass between the electrodes through which a direct current is flowing. In this embodiment, forward scattered light intensity is used as the size information.

Here, the spurious particles refer to the particles that affect the detection of the aggregation of the carrier particles. For example, when the specimen is a whole blood, the spurious particles may be blood cell components such as erythrocytes and platelets that are present in the blood, fragments of the blood cell components such as fractured erythrocytes and fractured platelets, fat particles, and the like.

Here, the degree of aggregation of the carrier particles refers to the degree of aggregation of the carrier particles based on the antigen-antibody reaction.

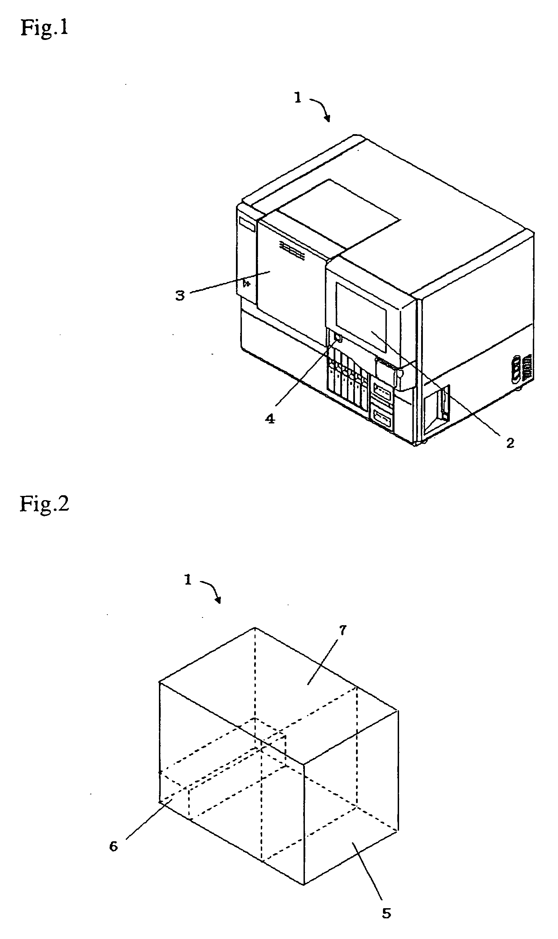

Fig. 1 shows an outlook of an immunoassay apparatus 1. A liquid crystal touch panel 2 for inputting various settings and outputting measurement results for display, a measuring sample preparing section cover 3, and a start switch 4 are disposed on the front of immunoassay apparatus 1.
Fig. 2 shows an internal configuration of immunoassay apparatus 1. A controlling section 5 that controls the operation of the apparatus and the analyzing process is disposed in a space on the right side of immunoassay apparatus 1. A measuring section 6 for detecting a signal from a measuring sample is disposed in a space on the lower left side of immunoassay apparatus 1. Also, a measuring sample preparing section 7 for preparing a measuring sample is disposed in the rest of the space.

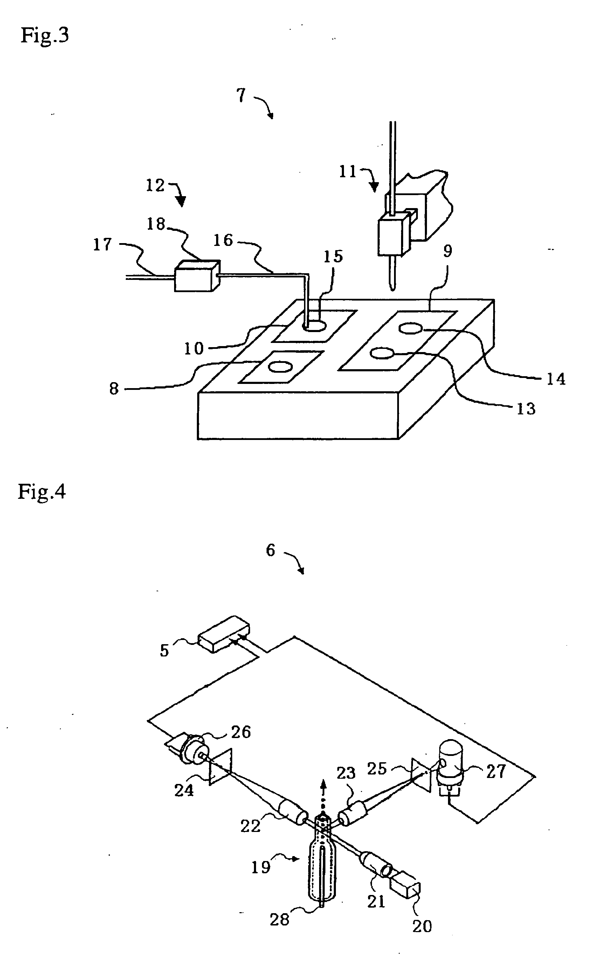

Fig. 3 is a view illustrating measuring sample preparing section 7. Measuring sample preparing section 7 is comprised of a specimen setting section 8, a reagent setting section 9, a reaction section 10, a dispensing device 11, and a liquid transporting device 12. An operator opens the aforementioned measuring sample preparing section cover 3 of Fig. 1 to set a specimen container containing a specimen into specimen setting section 8 and to set a container 13 containing a reaction buffer solution and a container 14 containing a carrier particle suspension into a reagent setting section 9. A micro test tube 15 is set in reaction section 10, where the specimen is mixed with the reaction buffer solution and the carrier particle suspension for preparation of a measuring sample. Here, though not illustrated in the drawings, reaction section 10 is provided with a temperature regulating mechanism for maintaining the solution in micro test tube 15 at a constant temperature and a stirring mechanism for stirring the solution in micro test tube 15. A dispensing device 11 is adapted to suck and eject a predetermined amount of liquid through the tip end thereof, and also dispensing device 11 is adapted to be movable upwards, downwards, rightwards, and leftwards by a driving device (not illustrated). Liquid transporting device 12 is comprised of a suction tube 16 for sucking a measuring sample, a liquid transporting pipe 17 for transporting the measuring sample sucked from suction tube 16 to measuring section 6 illustrated in Fig. 4, and a pump 18 for sucking the measuring sample and transporting the measuring sample to measuring section 6. Suction tube 16 is inserted into micro test tube 15 set in reaction section 10 so as to suck a predetermined amount of the measuring sample. The sucked measuring sample is transported to measuring section 6 through liquid transporting pipe 17.

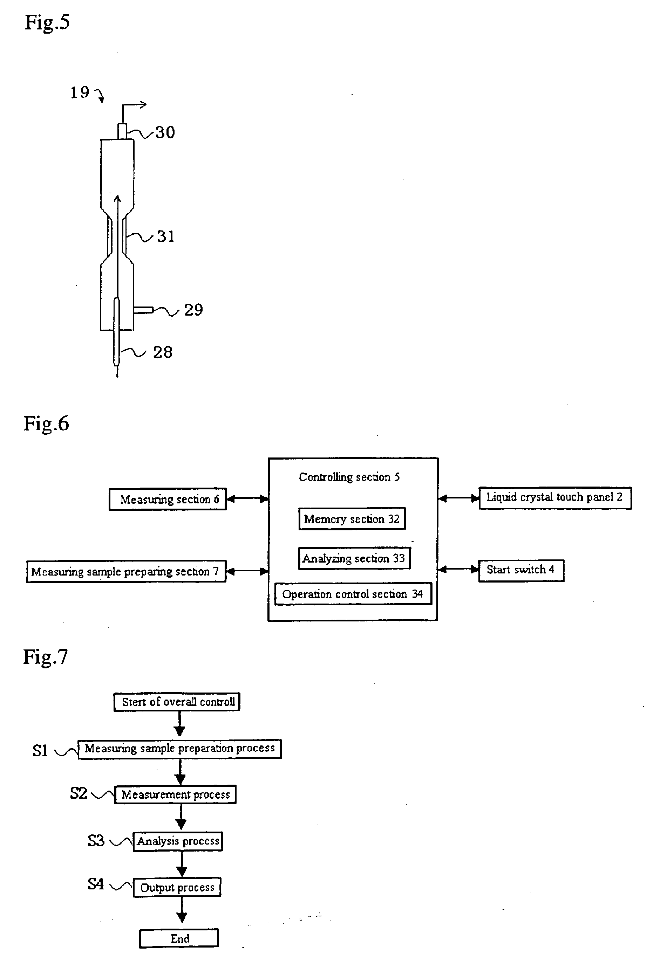

Fig. 4 is a view illustrating measuring section 6. Measuring section 6 is provided with a sheath flow cell 19, a laser light source 20, a condenser lens 21, converging lenses 22, 23, pin holes 24, 25, a photodiode 26, and a photomultiplier tube 27. Sheath flow cell 19 is for allowing the measuring sample prepared in the aforementioned measuring sample preparing section 7 of Fig. 3 to flow therethrough. Also, referring to Fig. 5, sheath flow cell 19 is provided with a sample nozzle 28 for jetting the measuring sample liquid upwards towards a narrow through-hole section 31, a sheath liquid supplying inlet 29, and an exhaust liquid outlet 30. Converging lens 22 collects optical information such as forward scattered light obtained from each particle in the measuring sample that has received a laser beam. Converging lens 23 collects optical information such as side scattered light obtained from each particle in the measuring sample that has received a laser beam. Photodiode 26 receives and performs photoelectric conversion on the forward scattered light to output an electric signal. Photomultiplier tube 27 receives and performs photoelectric conversion on the side scattered light to output an electric signal. The output signals are each sent to controlling section 5.
Fig. 6 is a view illustrating a configuration of controlling section 5 and a relationship between controlling section 5 and each section of the apparatus. Controlling section 5 includes a micro computer having a central processing unit (CPU) and a memory device such as a ROM or RAM and a circuit for processing the signals sent from measuring section 6. Controlling section 5 functions as a memory section 32, an analyzing section 33, and an operation controlling section 34. Memory section 32 memorizes analyzing programs for analyzing the signals obtained from particles in the measuring sample and controlling programs for controlling the operation of each section in the apparatus. Further, memory section 32 memorizes data of the signals detected by measuring section 6 and the results of processing by the analyzing programs. Analyzing section 33 analyzes the signals detected by measuring section 6 in accordance with the analyzing programs and creates data related to each particle contained in the measuring sample liquid. The data created in analyzing section 33 are output to liquid crystal touch panel 2. Operation controlling section 34 controls the operation of each section in the apparatus in accordance with the controlling programs memorized in memory section 32.

Hereinafter, the operation of the apparatus will be described in detail.

First, an operator sets a specimen and reagents for measurement to predetermined positions in measuring sample preparing section 7. The specimen can be set into specimen setting section 8 of the aforementioned measuring sample preparing section 7 of Fig. 3 by opening the aforementioned measuring sample preparing section cover 3 of Fig. 1. Further, a container 13 containing the reaction buffer solution and a container 14 containing the carrier particle suspension can be each set into reagent setting section 9 of measuring sample preparing section 7.

When the specimen and the reagents are set in this manner and a start switch 4 is pressed, an overall control is started. Fig. 7 is a flowchart showing the flow of the overall control by the controlling programs. When the start switch 4 is pressed, the steps S1 (measuring sample preparation process), S2 (measurement process), S3 (analysis process), and S4 (output process) are successively executed. Measuring sample preparing section 7, measuring section 6, and analyzing section 33 are controlled by the controlling programs, whereby a series of operations are automatically carried out. The above-mentioned steps S1, S2, S3, and S4 will be described below.

### S1 (measuring sample preparation process)

An operation of measuring sample preparing section 7 in measuring sample preparation will be described with reference to Fig. 3. First, dispensing device 11 sucks a specimen from a specimen container set in specimen setting section 8, and dispenses 10 *µ* L into micro test tube 15 set in reaction section 10. Next, dispensing device 11 sucks a reaction buffer solution from container 13 set in reagent setting section 9, and dispenses 80 *µ* L into micro test tube 15 set in reaction section 10. Further, dispensing device 11 sucks a carrier particle suspension from container 14 set in reagent setting section 9, and dispenses 10 *µ* L into micro test tube 15 set in reaction section 10. Thereafter, reaction section 10 stirs the mixture for 15 minutes while maintaining micro test tube 15 at a temperature of 45°C. This prepares a measuring sample in micro test tube 15. When the measuring sample is prepared, the measuring sample is sucked from micro test tube 15 of reaction section 10 by liquid transporting device 12, and is sent to sheath flow cell 19 of measuring section 6.

### S2 (measurement process)

An operation of measuring section 6 in the measurement will be described with reference to Figs. 4 and 5. The measuring sample prepared in measuring sample preparing section 7 is guided to sheath flow cell 19, and the measuring sample is ejected into the sheath flow cell through sample nozzle 28. Simultaneously with this, a sheath liquid is ejected into the sheath flow cell through sheath liquid supplying inlet 29. By this, the measuring sample liquid is surrounded by the sheath liquid within the sheath flow cell, and is further narrowed down by narrow through-hole section 31 to flow. By narrowing the flow of the measuring sample liquid to the same degree as the particle size, the particles contained in the measuring sample liquid are arranged in one line to flow through the narrow through-hole section.

A laser beam emitted from laser light source 20 is narrowed by condenser lens 21 and is radiated onto the sample stream flowing through narrow through-hole section 31. The forward scattered light emitted from each particle in the measuring sample that has received the laser beam is converged by converging lens 22 to pass through pin hole 24. The side scattered light is converged by converging lens 23 to pass through pin hole 25. Then, the forward scattered light is received and undergoes photoelectric conversion by photodiode 26, and is output as a forward scattered light signal. The side scattered light is received and undergoes photoelectric conversion by photomultiplier tube 27, and is output as a side scattered light signal. Each of the output signals is sent to controlling section 5, and is memorized into memory section 32 as data of individual particles.

### S3 (analysis process)

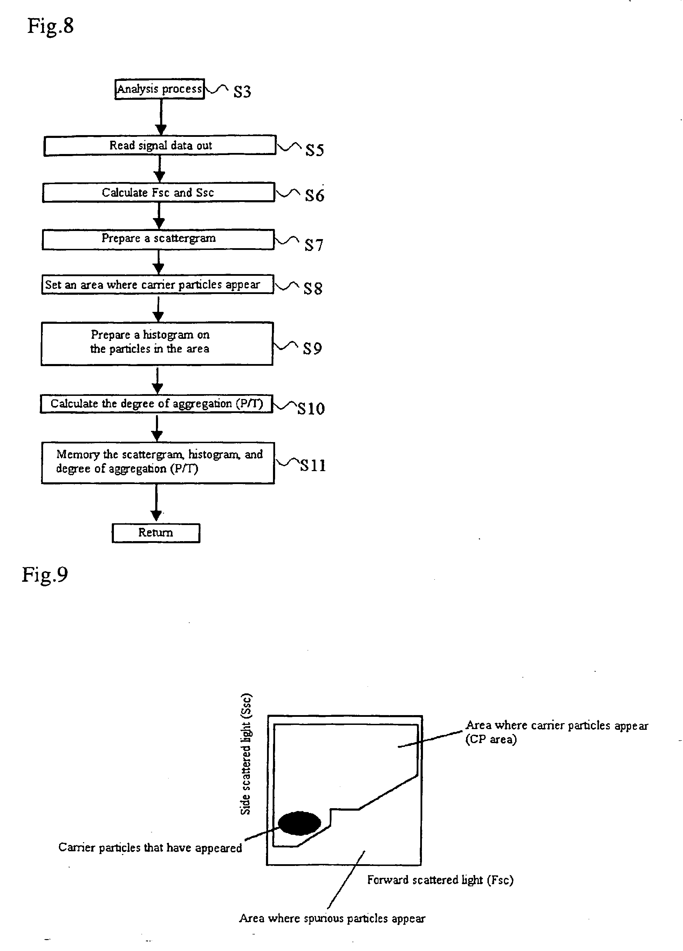

When a forward scattered light signal and a side scattered light signal are detected by the measurement process of S2, analyzing section 33 then analyzes each signal in accordance with the analyzing programs. An operation of the analyzing programs in the analysis process will be described with reference to the flowchart of Fig. 8. Each step in the flowchart is as follows.

S5: The data of the forward scattered light signal and the side scattered light signal detected from the measuring sample are read out from memory section 32. Then, the procedure goes to S6.

S6: The forward scattered light intensity (Fsc) and the side scattered light intensity (Ssc) are calculated on the basis of the forward scattered light signal and the side scattered light signal obtained from each particle in the measuring sample. Subsequently, the procedure goes to S7.

S7: A scattergram is prepared using the Fsc and the Ssc of each particle calculated in S6 as parameters. This is carried out as follows. First, two-dimensional coordinates are developed taking the Fsc and the Ssc as coordinate axes, and then the pair of coordinates corresponding to each particle in the measuring sample is plotted on the basis of the Fsc and the Ssc calculated in S6. In this manner, a scattergram is prepared using the Fsc and the Ssc as parameters. Then, the procedure goes to S8.

S8: An area where carrier particles appear (this will be hereafter referred to as CP area) is set on the prepared scattergram. The manner in which the CP area is set on the scattergram is illustrated in Fig. 9. In the scattergram, the longitudinal axis represents the side scattered light intensity (Ssc), and the horizontal axis represents the forward scattered light intensity (Fsc). The Fsc is the information that reflects the size of the particle, so that the size of the particle increases according as the particle is located more to the right side on the scattergram. The Ssc is the information that reflects the density of the particle, so that the density of the particle increases according as the particle is located more to the upper side on the scattergram. The carrier particles tend to have a higher density than the spurious particles. Therefore, by using a scattergram having the Ssc as a parameter, the carrier particles can be differentiated from the spurious particles. Here, the CP area that is set for differentiating the carrier particles is empirically determined by measuring the measuring samples prepared using specimens containing only the analyte and the measuring samples prepared using specimens containing the analyte and the spurious particles. Thus, the carrier particles contained in the measuring samples appear in the CP area, whereas the spurious particles appear outside of the CP area. Here, the CP area, which is memorized in memory section 32, is read out by the analyzing programs in S8 to be applied onto the scattergram. Then, the procedure goes to S9.

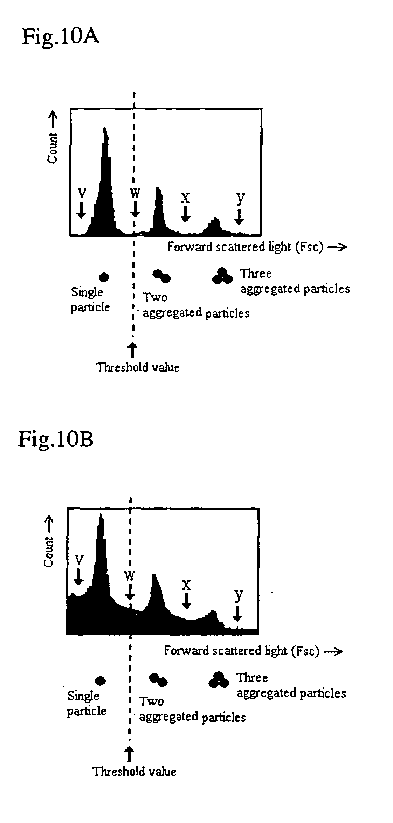

S9: A histogram is prepared with respect to the carrier particles appearing in the CP area that is set on the scattergram. Fig. 10A is one example of a histogram that is prepared with the use of the Fsc of the carrier particles appearing in the CP area. The longitudinal axis represents the number of particles (count), and the horizontal axis represents the Fsc. Then the procedure goes to S 10.

S 10: A degree of aggregation is calculated on the basis of the histogram prepared in S9. Here, first the single particles are differentiated from the aggregated particles on the basis of the histogram prepared in S9. Then the number of single particles (M) and the number of aggregated particles (P) are counted. Further, the total number of particles (T) is determined which is the sum of M and P, so as to calculate P/T as the degree of aggregation. Subsequently, the procedure goes to S11.

S11: The data of the scattergram prepared in S7 and S8, the histogram prepared in S9, and the degree of aggregation calculated in S 10 are memorized.

### S4 (output process)

The data of the scattergram prepared in S7 and S8, the histogram prepared in S9, and the degree of aggregation calculated in S 10 are output to liquid crystal touch panel 2 for display.

The above is the flow chart of the measurement in this embodiment.

Figs. 10A and 10B show one example of a histogram obtained by measurement in immunoassay apparatus 1 of this embodiment using whole blood as a specimen. Fig. 10A is a histogram prepared with respect to the carrier particles appearing in the CP area. On the other hand, Fig. 10B is a histogram prepared with respect to the carrier particles appearing in the CP area and the spurious particles appearing outside of the CP area.

As will be understood from Fig. 10A, the detected particles are distributed at positions corresponding to the size of the carrier particles, such as single particles, two aggregated particles, and three aggregated particles. As will be denoted with v, w, x, and y in Fig. 10A, substantially no particles are distributed at a site having a smaller size than the single particles, at a site between the single particles and the two aggregated particles, at a site between the two aggregated particles and the three aggregated particles, and at a site having a larger size than the three aggregated particles. In such a histogram, a threshold value is set between the forward scattered light intensity corresponding to the size of the single particles and the forward scattered light intensity corresponding to the size of the two aggregated particles. By identifying the particles distributed in a range smaller than the above-mentioned threshold value as single particles and the particles distributed in a range larger than the above-mentioned threshold value as aggregated particles, the number of single particles and the number of aggregated particles can be counted.

On the other hand, the histogram shown in Fig. 10B contains not only the carrier particles but also the spurious particles in the whole blood specimen, so that particles are distributed at the sites of v, w, x, and y where particles should not inherently be distributed. For this reason, even if the number of single particles and the number of aggregated particles are calculated on the basis of the histogram that is affected by the spurious particles as in Fig. 10B, the calculated values may not be accurate, and the degree of aggregation of the carrier particles calculated from the number of single particles and the number of aggregated particles may not be an accurate one.

As will be understood from comparison between Fig. 10A and Fig. 10B, by differentiating the carrier particles from the spurious particles and preparing a histogram based on the carrier particles alone, the influence of the spurious particles can be efficiently eliminated, so that an accurate degree of aggregation of the carrier particles can be calculated.

### (Measurement example)

An example of a result of the analysis of a specimen using immunoassay apparatus 1 described above will be shown.

For the measurement in this example, RANREAM HBsAg manufactured by Sysmex Co., Ltd. was used. This is a reagent kit for the measurement of HBs antigen, and is constituted of HBsAg latex reagent, HBsAg buffer solution, HBsAg specimen diluting liquid, and HBsAg calibrator. In this example, HBsAg latex reagent was used as a carrier particle suspension, and HBsAg buffer solution was used as a reaction buffer solution. The HBsAg latex reagent is a suspension of latex particles on which an anti-HBs antibody is immobilized. Here, the HBs antigen is a surface antigen of B-type hepatitis virus (HBV), so that one can examine whether the specimen is in a state infected with HBV or not by measurement using the reagent for HBs antigen measurement.

Also, in this example, an HBsAg-negative whole blood collected from a human being and an HBsAg-positive whole blood collected from a human being were used respectively as specimens.

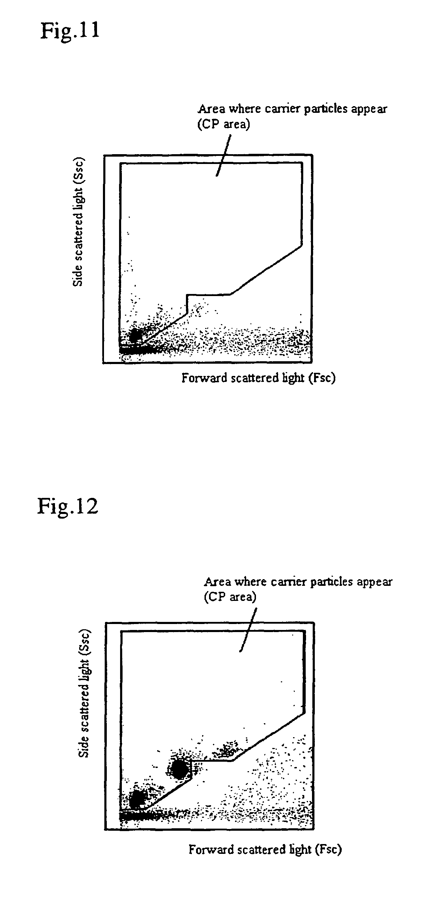

Figs. 11 and 12 show scattergrams obtained by the measurement using the above reagent and the specimens. Fig. 11 is a scattergram obtained by using the whole blood that is HBsAg negative as a specimen, and Fig. 12 is a scattergram obtained by using the whole blood that is HBsAg positive as a specimen. In each of the scattergrams, the dots corresponding to the latex particles appear in the CP area, and the dots corresponding to the spurious particles appear outside of the CP area. Here, the spurious particles contained in the specimens used in this example are mainly platelets.

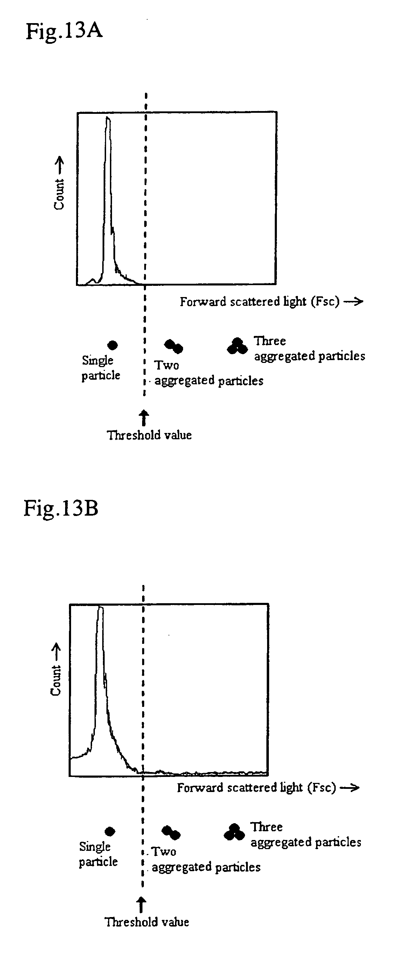

Figs. 13A and 13B are histograms prepared with respect to the particles that appear on the scattergram (Fig. 11) when the HBsAg-negative whole blood is used. Fig. 13A is a histogram prepared with respect to the carrier particles that appear in the CP area of Fig. 11. Fig. 13B is a histogram prepared with respect to the carrier particles that appear in the CP area of Fig. 11 and the spurious particles that appear outside of the CP area. From these, it will be understood that the influence of the spurious particles, such as the overall rise of base line that is seen in the histogram of Fig. 13B, is removed in the histogram of Fig. 13A.

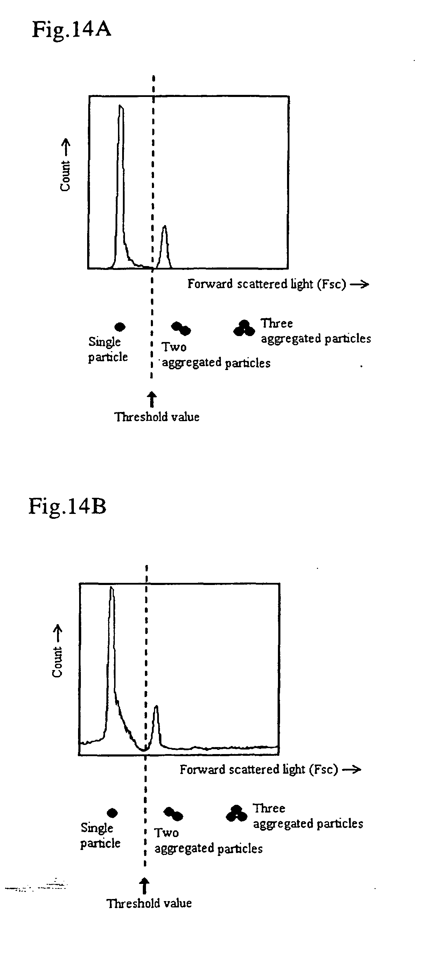

Figs. 14A and 14B are histograms prepared with respect to the particles that appear on the scattergram (Fig. 12) when the HBsAg-positive whole blood is used. Fig. 14A is a histogram prepared with respect to the carrier particles that appear in the CP area of Fig. 12. Fig. 14B is a histogram prepared with respect to the carrier particles that appear in the CP area of Fig. 12 and the spurious particles that appear outside of the CP area. From these, it will be understood that the influence of the spurious particles, such as the overall rise of base line that is seen in the histogram of Fig. 14B, is removed in the histogram of Fig. 14A.

Next, the degree of aggregation (P/T) calculated on the basis of the aforesaid histograms shown in Figs. 13A and 13B and Figs. 14A and 14B will be shown in the following Table 1.

**Table 1**

| | Degree of aggregation (P/T %) |
|---|---|
| 1 3 - A | 0.49 |
| 1 3 - B | 1.24 |
| 1 4 - A | 12.50 |
| 1 4 - B | 14.65 |

The item 13-A in Table 1 is the degree of aggregation (P/T %) calculated on the basis of the histogram (Fig. 13A) prepared with respect to the carrier particles that appear in the CP area among the histograms obtained using the HBsAg-negative whole blood as a specimen. The item 13-B in Table 1 is the degree of aggregation (P/T %) calculated on the basis of the histogram (Fig. 13B) prepared with respect to the carrier particles that appear in the CP area and the spurious particles that appear outside of the CP area among the histograms obtained using the HBsAg-negative whole blood as a specimen. The item 14-A in Table 1 is the degree of aggregation (P/T %) calculated on the basis of the histogram (Fig. 14A) prepared with respect to the carrier particles that appear in the CP area among the histograms obtained using the HBsAg-positive whole blood as a specimen. The item 14-B in Table 1 is the degree of aggregation (P/T %) calculated on the basis of the histogram (Fig. 14B) prepared with respect to the carrier particles that appear in the CP area and the spurious particles that appear outside of the CP area among the histograms obtained using the HBsAg-positive whole blood as a specimen.

In Table 1, the degree of aggregation of 13-B shows a higher value than the degree of aggregation of 13-A. Similarly, the degree of aggregation of 14-B shows a higher value than the degree of aggregation of 14-A.

From the above, it will be understood that, when the degree of aggregation is calculated on the basis of the histogram prepared with respect to the carrier particles that appear in the CP area and the spurious particles that appear outside of the CP area, the degree of aggregation is affected by the spurious particles, so that the calculated degree of aggregation has a higher value than the actual degree of aggregation.

Hereinafter, calculation of the concentration of an analyte will be described.

The concentration of an analyte contained in a specimen can be determined by using a calibration line that is prepared on the basis of the degree of aggregation of the carrier particles obtained by measuring beforehand a specimen that contains the analyte at a known concentration. Therefore, in this example, a calibration line is prepared using the HBsAg specimen diluting liquid and the HBsAg calibrator of RANREAM HBsAg manufactured by Sysmex Co., Ltd., and the concentration of HBs antigen was calculated on the basis of the degree of aggregation (Table 1) calculated from the histograms of Figs. 13A and 13B and Figs. 14A and 14B. The HBsAg specimen diluting liquid is a liquid that does not contain an HBs antigen. The HBsAg calibrator is a solution that contains an HBs antigen, and the antigen concentration is adjusted in six stages. Therefore, the HBsAg specimen diluting liquid and the HBsAg calibrator were used as a specimen for preparing a calibration line of the antigen concentration in a sum of seven stages, whereby the calibration line was prepared. The concentration of the HBs antigen calculated on the basis of this calibration line is shown in the following Table 2.

**Table 2**

| | Concentration of HBs antigen (U/mL) |
|---|---|
| 1 3 - a | 0 |
| 1 3 - b | 0.4 |
| 1 4 - a | 8.5 |
| 1 4 - b | 14.0 |

The item 13-a in Table 2 is the concentration (U/mL) of the HBs antigen calculated on the basis of the degree of aggregation of 13-A of Table 1. The item 13-b in Table 2 is the concentration (U/mL) of the HBs antigen calculated on the basis of the degree of aggregation of 13-B of Table 1. The item 14-a in Table 2 is the concentration (U/mL) of the HBs antigen calculated on the basis of the degree of aggregation of 14-A of Table 1. The item 14-b in Table 2 is the concentration (U/mL) of the HBs antigen calculated on the basis of the degree of aggregation of 14-B of Table 1.

In Table 2, compared with the concentration of the HBs antigen of 13-a being zero (U/mL), the concentration of the HBs antigen of 13-b is 0.4 (U/mL), showing a higher value than the concentration of the HBs antigen of 13-a. Similarly, compared with the concentration of the HBs antigen of 14-a being 8.5 (U/mL), the concentration of the HBs antigen of 14-b is 14.0 (U/mL), showing a higher value than the concentration of the HBs antigen of 14-a. From the above, it will be understood that the spurious particles appearing outside of the CP area give a large influence on the concentration of the HBs antigen.

In other words, the results of Figs. 13A and 13B, Figs. 14A and 14B, and Tables 1 and 2 show that setting a CP area by a method such as described above and calculating the degree of aggregation and the concentration of HBs antigen with respect to the carrier particles appearing in the CP area are extremely effective in removing the influence of spurious particles that guides to an erroneous measurement results.

This immunoassay apparatus 1 detects internal information of each particle in a measuring sample, and differentiates the carrier particles from the spurious particles on the basis of the internal information. Therefore, in immunoassay apparatus 1, the degree of aggregation of the carrier particles can be determined accurately by removing the influence of the spurious particles.

Here, in this embodiment, a whole blood collected from a human being is used as a specimen; however, the present invention is not limited to this alone. Instead of whole blood, it is possible to use serum and plasma as a specimen. Further, blood or urine that contains spurious particles such as blood cells, fragments of blood cell components, bacteria, and fat particles can be used as a specimen as well.

In this embodiment, the HBsAg buffer solution of RANREAM HBsAg manufactured by Sysmex Co., Ltd. is used as a reaction buffer solution; however, the reaction buffer solution that can used is not limited to this alone. For example, a solution having a buffer function around about pH 6 to 8.5 can be used as a reaction buffer solution. The kind of buffer solution may be, for example, phosphate buffer solution or Tris-hydrochloric acid buffer solution. Further, a substance for restraining nonspecific reaction, a sensitizer, and others can be added to the reaction buffer solution in accordance with the needs.

In this embodiment, the latex particles contained in the HBsAg latex reagent of RANREAM HBsAg manufactured by Sysmex Co., Ltd. are used as the carrier particles; however, the present invention is not limited to this alone. For example, any carrier particles on which an antibody or an antigen against the analyte is immobilized can be used. A suitable size of the particles is a diameter of about 0.1 to 1.0 *µ* m. A method of sensitizing the carrier particles with an antibody or an antigen may be a method conventionally known in the field of art. For example, the method may be the physical absorption method, the chemical bonding method, or the like. The antibody or antigen for sensitizing the carrier particles is not particularly limited as long as it can be detected using antigen-antibody reaction.

In this embodiment, an anti-HBs antigen is detected as an analyte by the immunoassay apparatus; however, the analyte of the present invention is not limited to this alone. Any analyte that can be detected by immunoassay using the carrier particles in the field of art can be detected as an analyte. For example, the analyte may be carcinoembryonic antigen (CEA), prostate gland specific antigen (PSA), anti-HCV antibody, insulin, or ferritin (FRN).

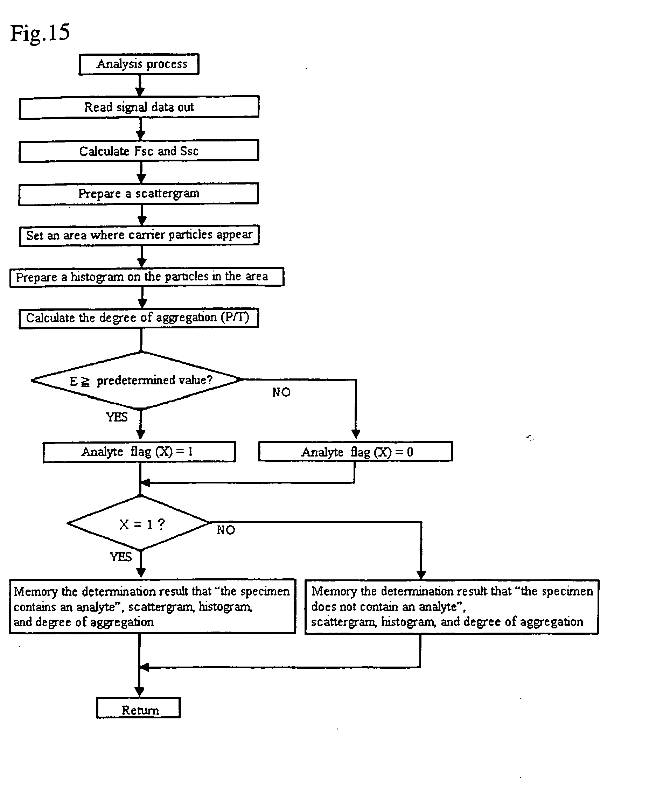

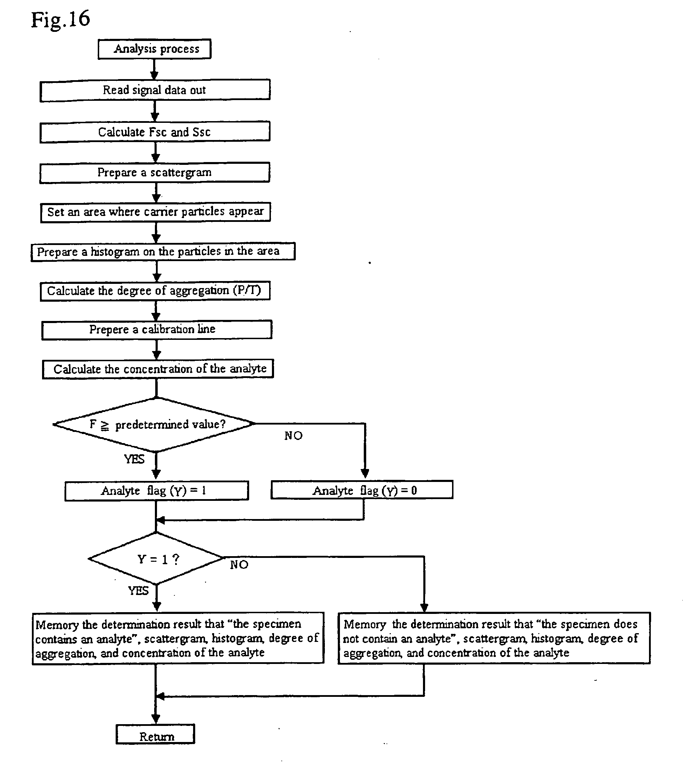

Immunoassay apparatus 1 of this embodiment calculates the degree of aggregation in the analyzing step. However the present invention is not limited to this alone. For example, an analyzing program of calculating the degree of aggregation and determining whether or not the analyte is contained in the specimen such as shown in Fig. 15 may be memorized in memory section 32 of Fig. 6, and an analysis process may be carried out by analyzing section 33 in accordance with this analyzing program. One method of determining whether or not the analyte is contained in the specimen on the basis of the calculated degree of aggregation may be a method of setting beforehand a predetermined value with respect to the degree of aggregation and determining whether or not the analyte is contained in the specimen by comparing the calculated degree (E) of aggregation with this predetermined value. Also, an analyzing program of calculating the degree of aggregation, preparing a calibration line, calculating the concentration of an analyte contained in a specimen, and determining whether or not the analyte is contained in the specimen such as shown in Fig. 16 may be memorized in memory section 32 of Fig. 6, and an analysis process may be carried out by analyzing section 33 in accordance with this analyzing program. One method of determining whether or not the analyte is contained in the specimen on the basis of the concentration of the analyte may be a method of setting beforehand a predetermined value with respect to the concentration of the analyte and determining whether or not the analyte is contained in the specimen by comparing the calculated concentration (F) of the analyte with this predetermined value.

In measuring section 6 of this embodiment, converging lens 23, pinhole 25, and photomultiplier tube 27 are disposed so as to receive the side scattered light that is scattered in the direction perpendicular to the direction of the progressing laser light emitted from the laser source; however, the present invention is not limited to this alone. They can be disposed in any direction relative to the direction of the progressing laser light as long as they are disposed at positions where they can receive the scattered light that reflects the internal information.

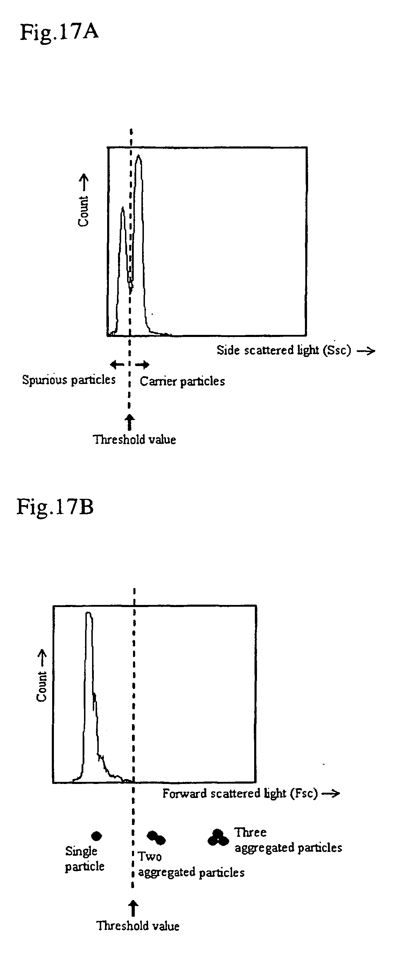

In this embodiment, the carrier particles are differentiated from the spurious particles on the basis of the two pieces of information, i.e. the side scattered light intensity and the forward scattered light intensity; however, the present invention is not limited to this alone. For example, the carrier particles can be differentiated from the spurious particles on the basis of the information of side scattered light intensity alone. One such method may be the following method. First, referring to Fig. 17A, a histogram is prepared taking the number of particles (count) as the longitudinal axis and the side scattered light intensity as the horizontal axis. As described above, the carrier particles tend to have a higher density than the spurious particles. Therefore, in a histogram such as shown in Fig. 17A, the spurious particles appear at positions corresponding to a smaller side scattered light intensity, whereas the carrier particles appear at positions corresponding to a larger side scattered light intensity. For this reason, by setting a threshold value between the side scattered light intensity corresponding to the carrier particles and the side scattered light intensity corresponding to the spurious particles, the particles distributed in a range smaller than the above-mentioned threshold value can be identified as the spurious particles, and the particles distributed in a range larger than the above-mentioned threshold value can be identified as the carrier particles. Here, the degree of aggregation of the identified carrier particles can be calculated on the basis of a histogram (Fig. 17B) taking the number of particles (count) as the longitudinal axis and the forward scattered light intensity as the horizontal axis, in a manner similar to that of the above-described embodiment.

In this embodiment, the side scattered light intensity that reflects the density is used as the internal information, and the forward scattered light intensity is used as the size information. However, the present invention is not limited to this alone. For example, high frequency resistance can be used as the internal information, and direct current resistance can be used as the size information. In this case, a narrow through-hole section through which the particles contained in a measuring sample pass is disposed in the measuring section, and electrodes are disposed on both sides of the narrow through-hole section. A high frequency current and a direct current are applied between these electrodes so as to detect the change in the high frequency resistance and the change in the direct current resistance that occur when the particles pass through the narrow through-hole section.

## Claims

1. An immunoassay apparatus comprising:
(a) a measuring sample preparing section for preparing a sample by mixing a specimen with carrier particles on which an antibody or an antigen against an analyte is immobilized;
(b) a detector for detecting internal information and size information from particles contained in the measuring sample, which internal information is selected from the group consisting of side scattered light and high frequency current resistance; and
(c) a controller which identifies the carrier particles on the basis of the obtained internal information and calculates a degree of aggregation of the identified carrier particles on the basis of the obtained size information.

2. The immunoassay apparatus according to claim 1, wherein said controller identifies the carrier particles on the basis of the internal information and the size information.

3. The immunoassay apparatus according to claim 1 or 2, wherein said size information includes forward scattered light or direct current resistance.

4. An immunoassay apparatus comprising:
(a) a measuring sample preparing section for preparing a measuring sample by mixing a specimen with carrier particles on which an antibody or an antigen against an analyte is immobilize;
(b) a detector for detecting side scattered light and forward scattered light from particles contained in the measuring sample; and
(c) a controller which identifies the carrier particles on the basis of the detected side scattered light and calculates a degree of aggregation of the identified carrier particles on the basis of the detected forward scattered light.

5. The immunoassay apparatus according to claim 4, wherein said controller identifies the carrier particles on the basis of the side scattered light and the forward scattered light.

6. The immunoassay apparatus according to claim 1, 2, 3, 4 or 5, wherein said specimen is a whole blood.

7. The immunoassay apparatus according to claim 1, 2, 3, 4, 5 or 6, wherein said controller further calculates a concentration of the analyte contained in the specimen on the basis of said degree of aggregation.

8. The immunoassay apparatus according to claim 1, 2, 3, 4, 5 or 6, wherein-said controller further determines whether or not the analyte is contained in the specimen on the basis of said degree of aggregation.

9. Amethodof identifying carrier particles in an immunoassay, comprising the steps of:
(a) preparing a measuring sample by mixing a specimen with carrier particles on which an antibody or an antigen against an analyte is immobilized;
(b) detecting internal information from particles contained in the prepared measuring sample, which internal information is selected from the group consisting of side scattered light and high frequency current resistance; and
(c) identifying the carrier particles on the basis of the obtained internal information.

10. An immunoassay method comprising the steps of:
(a) preparing an measuring sample by mixing a specimen with carrier particles on which an antibody or an antigen against an analyte is immobilized;
(b) detecting internal information and size information from particles contained in the measuring sample, which internal information is selected from the group consisting of side scattered light and high frequency current resistance;
(c) identifying the carrier particles on the basis of the obtained internal information; and
(d) calculating a degree of aggregation of the carrier particles identified by said step (c) on the basis of the obtained size information.

11. The immunoassay method according to claim 10, wherein said step (c) identifies the carrier particles on the basis of said internal information and the size information of the particles.

12. An immunoassay method comprising the steps of:
(a) preparing an measuring sample by mixing a specimen with carrier particles on which an antibody or an antigen against an analyte is immobilized;
(b) detecting side scattered light and forward scattered light from particles contained in the measuring sample;
(c) identifying the carrier particles on the basis of the detected side scattered light; and
(d) calculating a degree of aggregation of the carrier particles identified by said step (c) on the basis of the detected forward scattered light.

13. The immunoassay method according to claim 12, wherein said step (c) identifies the carrier particles on the basis of said side scattered light and said forward scattered light.

14. The immunoassay method according to claim 10, 11, 12 or 13, further comprising a step of calculating a concentration of the analyte contained in the specimen on the basis of said degree of aggregation.

15. The immunoassay method according to claim 10, 11, 12 or 13, further comprising a step of determining whether or not the analyte is contained in the specimen on the basis of said degree of aggregation.

## Patentansprüche

1. Immuntestvorrichtung, umfassend:
(a) einen Messuntersuchungsproben-Präparationsabschnitt zum Präparieren einer Untersuchungsprobe durch Mischen einer Probe mit Trägerpartikeln, auf denen ein Antikörper oder ein Antigen gegen einen Analyt immobilisiert ist;
(b) einen Detektor zum Detektieren einer internen Information und Größeninformation von Partikeln, die in der Messuntersuchungsprobe enthalten sind, welche interne Information aus der Gruppe ausgewählt ist, die aus Seitenstreulicht und Hochfrequenzstromwiderstand besteht; und
(c) eine Steuerung, die die Trägerpartikel auf der Basis der erhaltenen internen Information identifiziert und ein Aggregationsmaß der identifizierten Trägerpartikel auf der Basis der erhaltenen Größeninformation berechnet.

2. Immuntestvorrichtung nach Anspruch 1, wobei die Steuerung die Trägerpartikel auf der Basis der internen Information und der Größeninformation identifiziert.

3. Immuntestvorrichtung nach Anspruch 1 oder 2, wobei die Größeninformation Vorwärtsstreulicht oder Gleichstromwiderstand enthält.

4. Immuntestvorrichtung, umfassend:
(a) einen Messuntersuchungsproben-Präparationsabschnitt zum Präparieren einer Messuntersuchungsprobe durch Mischen einer Probe mit Trägerpartikeln, auf denen ein Antikörper oder ein Antigen gegen einen Analyt immobilisiert ist;
(b) einen Detektor zum Detektieren von Seitenstreulicht und Vorwärtsstreulicht von Partikeln, die in der Messuntersuchungsprobe enthalten sind; und
(c) eine Steuerung, die die Trägerpartikel auf der Basis des detektierten Seitenstreulichts identifiziert und ein Aggregationsmaß der identifizierten Trägerpartikel auf der Grundlage des detektierten Vorwärtsstreulichtes berechnet.

5. Immuntestvorrichtung nach Anspruch 4, wobei die Steuerung die Trägerpartikel auf der Basis des Seitenstreulichtes und des Vorwärtsstreulichtes identifiziert.

6. Immuntestvorrichtung nach Anspruch 1, 2, 3, 4 oder 5, wobei die Probe Vollblut ist.

7. Immuntestvorrichtung nach Anspruch 1, 2, 3, 4, 5, oder 6, wobei die Steuerung ferner eine Konzentration des Analyts, der in der Probe enthalten ist, auf der Basis des Aggregationsgrades berechnet.

8. Immuntestvorrichtung nach Anspruch 1, 2, 3, 4, 5 oder 6, wobei die Steuerung ferner auf der Grundlage des Aggregationsgrades bestimmt, ob der Analyt in der Probe enthalten ist oder nicht.

9. Verfahren zum Identifizieren von Trägerpartikeln in einem Immuntest, umfassend die folgenden Schritte:
(a) Präparieren einer Messuntersuchungsprobe durch Mischen einer Probe mit Trägerpartikeln, auf denen ein Antikörper oder ein Antigen gegen einen Analyt immobilisiert ist;
(b) Detektieren einer internen Information von Partikeln, die in der präparierten Messprobe enthalten sind, welche interne Information aus der Gruppe ausgewählt ist, die aus Seitenstreulicht und Hochfrequenzstromwiderstand besteht; und
(c) Identifizieren der Trägerpartikel auf der Basis der erhaltenen internen Information.

10. Immuntestverfahren, das die folgenden Schritte umfasst:
(a) Präparieren einer Messuntesuchungsprobe durch Mischen einer Probe mit Trägerpartikeln, auf denen ein Antikörper oder ein Antigen gegen einen Analyt immobilisiert ist;
(b) Detektieren einer internen Information und Größeninformation von Partikeln, die in der Messuntersuchungsprobe enthalten sind, welche interne Information aus der Gruppe ausgewählt, ist, die aus Seitenstreulicht und Hochfrequenzstromwiderstand besteht;
(c) Identifizieren der Trägerpartikel auf der Basis der erhaltenen internen Information; und
(d) Berechnen eines Aggregationsgrades der Trägerpartikel, die durch den Schritt (c) identifiziert wurden, auf der Basis der erhaltenen Größeninformation.

11. Immuntestverfahren nach Anspruch 10, wobei der Schritt (c) die Trägerpartikel auf der Basis der internen Information und der Größeninformation der Partikel identifiziert.

12. Immuntestverfahren, welches die folgenden Schritte umfasst:
(a) Präparieren einer Messuntersuchungsprobe durch Mischen einer Probe mit Trägerpartikeln, auf denen ein Antikörper oder ein Antigen gegen einen Analyt immobilisiert ist;
(b) Detektieren von Seitenstreulicht und Vorwärtsstreulicht von Partikeln, die in der Messuntersuchungsprobe enthalten sind;
(c) Identifizieren der Trägerpartikel auf der Basis des detektierten Seitenstreulichts; und
(d) Berechnen eines Aggregationsgrades der Trägerpartikel, die durch den Schritt (c) identifiziert wurden, auf der Basis des detektierten Vorwärtsstreulichtes.

13. Immuntestverfahren nach Anspruch 12, wobei der Schritt (c) die Trägerpartikel auf der Basis des Seitenstreulichtes und des Vorwärtsstreulichtes identifiziert.

14. Immuntestverfahren nach Anspruch 10, 11, 12 oder 13, ferner umfassend einen Schritt des Berechnens einer Konzentration des Analyts, der in der Probe enthalten ist, auf der Basis des Aggregationsgrades.

15. Immuntestverfahren nach Anspruch 10, 11, 12 oder 13, ferner umfassend einen Schritt des Bestimmens, ob der Analyt in der Probe enthalten ist oder nicht, auf der Basis des Aggregationsgrades.

## Revendications

1. Appareil d'immunoessai comprenant :
(a) une section de préparation d'échantillon de mesure pour la préparation d'un échantillon en mélangeant un spécimen avec des particules porteuses sur lesquelles un anticorps ou un antigène contre une substance à analyser est immobilisé ;
(b) un détecteur destiné à détecter des informations internes et des informations de dimension à partir de particules contenues dans l'échantillon de mesure, dont les informations internes sont sélectionnées du groupe composé de lumière à diffusion latérale et de résistance au courant haute fréquence ; et
(c) un dispositif de commande qui identifie les particules porteuses sur la base des informations internes obtenues et qui calcule un degré d'agrégation des particules porteuses identifiées sur la base des informations de dimension obtenues.

2. Appareil d'immunoessai selon la revendication 1, où ledit dispositif de commande identifie les particules porteuses sur la base des informations internes et des informations de dimension.

3. Appareil d'immunoessai selon la revendication 1 ou 2, où lesdites informations de dimension comprennent une lumière à diffusion avant ou une résistance au courant continu.

4. Appareil d'immunoessai comprenant :
(a) une section de préparation d'échantillon de mesure pour la préparation d'un échantillon de mesure en mélangeant un spécimen avec des particules porteuses sur lesquelles un anticorps ou un antigène contre une substance à analyser est immobilisé ;
(b) un détecteur destiné à détecter une lumière à diffusion latérale et une lumière à diffusion avant à partir de particules contenues dans l'échantillon de mesure ; et
(c) un dispositif de commande qui identifie les particules porteuses sur la base de la lumière à diffusion latérale et qui calcule un degré d'agrégation des particules porteuses identifiées sur la base de la lumière à diffusion avant détectée.

5. Appareil d'immunoessai selon la revendication 4, où ledit dispositif de commande identifie les particules porteuses sur la base de la lumière à diffusion latérale et de la lumière à diffusion avant.

6. Appareil d'immunoessai selon les revendications 1, 2, 3, 4 ou 5, où ledit spécimen est du sang total.

7. Appareil d'immunoessai selon les revendications 1, 2, 3, 4, 5 ou 6, où ledit dispositif de commande calcule en outre une concentration de la substance à analyser contenue dans le spécimen sur la base dudit degré d'agrégation.

8. Appareil d'immunoessai selon les revendications 1, 2, 3, 4, 5 ou 6, où ledit dispositif de commande détermine en outre si la substance à analyser est contenue dans le spécimen ou non sur la base dudit degré d'agrégation.

9. Procédé d'identification de particules porteuses dans un immunoessai, comprenant les étapes consistant à :
(a) préparer un échantillon de mesure en mélangeant un spécimen avec des particules porteuses sur lesquelles un anticorps ou un antigène contre une substance à analyser est immobilisé ;
(b) détecter des informations internes à partir de particules contenues dans l'échantillon de mesure préparé, dont les informations internes sont sélectionnées à partir du groupe composé de lumière à diffusion latérale et de résistance au courant haute fréquence ; et
(c) identifier les particules porteuses sur la base des informations internes obtenues.

10. Procédé d'immunoessai comprenant les étapes consistant à :
(a) préparer un échantillon de mesure en mélangeant un spécimen avec des particules porteuses sur lesquelles un anticorps ou un antigène contre une substance à analyser est immobilisé ;
(b) détecter des informations internes et des informations de dimension à partir de particules contenues dans l'échantillon de mesure, dont les informations internes sont sélectionnées à partir du groupe composé de lumière à diffusion latérale et de résistance au courant haute fréquence ;
(c) identifier les particules porteuses sur la base des informations internes obtenues ; et
(d) calculer un degré d'agrégation des particules porteuses identifiées par ladite étape (c) sur la base des informations de dimension obtenues.

11. Procédé d'immunoessai selon la revendication 10, où ladite étape (c) identifie les particules porteuses sur la base desdites informations internes et des informations de dimension des particules.

12. Procédé d'immunoessai comprenant les étapes consistant à :
(a) préparer un échantillon de mesure en mélangeant un spécimen avec des particules porteuses sur lesquelles un anticorps ou un antigène contre une substance à analyser est immobilisé ;
(b) détecter une lumière à diffusion latérale et une lumière à diffusion avant à partir de particules contenues dans l'échantillon de mesure ;
(c) identifier les particules porteuses sur la base de la lumière à diffusion latérale détectée ; et
(d) calculer un degré d'agrégation des particules porteuses identifiées par ladite étape (c) sur la base de la lumière à diffusion avant détectée.

13. Procédé d'immunoessai selon la revendication 12, où ladite étape (c) identifie les particules porteuses sur la base de ladite lumière à diffusion latérale et de ladite lumière à diffusion avant.

14. Procédé d'immunoessai selon les revendications 10, 11, 12 ou 13, comprenant en outre une étape destinée à calculer une concentration de la substance à analyser contenue dans le spécimen sur la base dudit degré d'agrégation.

15. Procédé d'immunoessai selon les revendications 10, 11, 12 ou 13, comprenant en outre une étape destinée à déterminer si la substance à analyser est contenue dans le spécimen ou non sur la base dudit degré d'agrégation.
